# EUROPEAN PATENT APPLICATION

(11) **EP 4 357 451 A1**
(43) Date of publication of application: **24.04.2024**
(21) Application number: 22824952.0
(22) Date of filing: 13.06.2022
(51) Int. Cl.: C12N 9/10, A23L 33/195, C12N 15/54, C12P 1/00, C12P 21/00

(54) **ENZYME AGENT CONTAINING TRANSGLUTAMINASE AND USE THEREOF**

(30) Priority: 14.06.2021 JP 2021098423
(71) Applicant: Amano Enzyme Inc., Nagoya-shi Aichi 460-8630 (JP)
(72) Inventor: TAKAHASHI, Tetsuya, Kakamigahara-shi, Gifu 509-0109 (JP); MATSUBARA, Hirotaka, Kakamigahara-shi, Gifu 509-0109 (JP); MATSUOKA, Tomohiro, Kakamigahara-shi, Gifu 509-0109 (JP); SAKAI, Kiyota, Kakamigahara-shi, Gifu 509-0109 (JP)
(74) Representative: Witthoff Jaekel Steinecke Patentanwälte PartG mbB
(86) International application number: PCT/JP2022/023617
(87) International publication number: WO 2022/264963

(57) **Abstract**

It is an object of the present invention to provide an enzyme agent containing a transglutaminase having high reactivity at a low temperature and the use thereof. According to the present invention, provided is an enzyme agent containing, as an active ingredient, a transglutaminase consisting of an amino acid sequence having a sequence identity of 90% or more to the amino acid sequence of SEQ ID NO: 1.

## Description

### Technical Field

The present invention relates to an enzyme agent (transglutaminase agent) containing a transglutaminase as an active ingredient, and the use thereof.

### Background Art

Transglutaminase (also referred to as "TG") is an enzyme that catalyzes the acyl transfer reaction of the γ-carboxylamide groups of glutamine residues present in a peptide chain, and when the ε-amino groups of lysine residues in a protein act as acyl receptors, transglutaminase forms an ε-(γ-Gln)-Lys cross-linking bond within or between the protein molecules.

Although various applications of transglutaminase have been developed for the use of protein processing, only a few enzymes have been put into practical use. Most of the enzymes used in food products are derived from Streptomyces sp. or related species thereof, and other than those, the transglutaminase derived from Bacillus sp. has been known. Patent Document 1 discloses a transglutaminase from Streptomyces sp., and Patent Document 2 discloses a transglutaminase derived from *Kutzneria albida.*

### Prior Art Documents

### Patent Documents

Patent Document 1: JP Patent Publication No. H4-108381 A (1992)
Patent Document 2: JP Patent Publication No. 2020-195397 A

### Summary of Invention

### Object to be Solved by the Invention

When transglutaminase is used for edible meat and seafood processing, the transglutaminase is required to react at a low temperature. However, existing transglutaminases have insufficient reactivity at a low temperature. It is an object of the present invention to provide an enzyme agent containing a transglutaminase having high reactivity at a low temperature and the use thereof.

### Means for Solving the Object

The present inventors have newly discovered a novel transglutaminase derived from *Longimycelium tulufanense,* and have identified the amino acid sequence thereof. The sequence identity between the amino acid sequence of the novel transglutaminase of the present invention and the amino acid sequence of the transglutaminase derived from Streptomyces sp. described in Patent Document 1 was 29%. Moreover, the sequence identity between the amino acid sequence of the novel transglutaminase of the present invention and the amino acid sequence of the transglutaminase derived from *Kutzneria albida* described in Patent Document 2 was 65%. Furthermore, the present inventors have measured the reactivity of the above-identified novel transglutaminase, and as a result, the inventors have found that this novel transglutaminase has high reactivity at a low temperature. The present invention has been completed based on the aforementioned findings.

According to the present invention, the following inventions are provided.
< 1 > An enzyme agent containing, as an active ingredient, a transglutaminase consisting of an amino acid sequence having a sequence identity of 90% or more to the amino acid sequence of SEQ ID NO: 1.
< 2 > The enzyme agent according to < 1 >, wherein the transglutaminase is derived from Longimycelium sp.
<3 > An enzyme agent containing, as an active ingredient, a transglutaminase derived from Longimycelium sp., in which the transglutaminase has a molecular weight of 28 to 30 kDa as measured by SDS-PAGE, and an enzyme activity of the transglutaminase at 20°C is 20% or more of the enzyme activity thereof at 50°C.
< 4 > The enzyme agent according to < 3 >, wherein an optimal pH is pH 6.5 to 7.5.
< 5 > The enzyme agent according to < 3 > or < 4 >, wherein the enzyme activity after a heat treatment at 50°C for 40 minutes is 60% or more of the enzyme activity before the heat treatment.
< 6 > The enzyme agent according to any one of < 3 > to < 5>, wherein the enzyme activity at pH 4 is 50% or more of the enzyme activity at pH 8.
< 7 > A method for modifying a protein or a peptide, containing allowing the enzyme agent according to any one of < 1 > to < 6 > to act on a substrate.
< 8 > A method for producing a food product, containing allowing the enzyme agent according to any one of < 1 > to < 6 > to act on a food product.
< 9 > A food product treated with the enzyme agent according to any one of < 1 > to < 6 >.
< 10 > A method for producing a transglutaminase consisting of an amino acid sequence having a sequence identity of 90% or more to the amino acid sequence of SEQ ID NO: 1, containing
   culturing Longimycelium sp. bacteria.
< 11 > A method for producing a transglutaminase, containing
   culturing a *Longimycelium tulufanense* NBRC107726 strain.

### Advantageous Effects of Invention

The enzyme agent containing the transglutaminase of the present invention has high reactivity at a low temperature, and is suitably used in the fields of food products and medical uses.

### Brief Description of Drawings

[Fig. 1] Fig. 1 shows the results obtained by confirming the molecular weight of a *Longimycelium tulufanense*-derived transglutaminase.
[Fig. 2] Fig. 2 shows a comparison made between the *Longimycelium tulufanense-*derived transglutaminase and a *S*. *mobaraensis-*derived transglutaminase in terms of optimal temperature.
[Fig. 3] Fig. 3 shows a comparison made between the *Longimycelium tulufanense*-derived transglutaminase and the *S*. *mobaraensis-*derived transglutaminase in terms of optimal pH.
[Fig. 4] Fig. 4 shows a comparison made between the *Longimycelium tulufanense-*derived transglutaminase and the *S*. *mobaraensis*-derived transglutaminase in terms of heat stability (50°C).
[Fig. 5] Fig. 5 shows a comparison made between the *Longimycelium tulufanense*-derived transglutaminase and the *S*. *mobaraensis*-derived transglutaminase in terms of pH stability.
[Fig. 6] Fig. 6 shows a comparison made between the *Longimycelium tulufanense*-derived transglutaminase and the *S*. *mobaraensis*-derived transglutaminase in terms of cross-linking reactivity.
[Fig. 7] Fig. 7 shows a comparison made between the *Longimycelium tulufanense*-derived transglutaminase and the *S*. *mobaraensis*-derived transglutaminase in terms of cross-linking activity to soybeans.
[Fig. 8] Fig. 8 shows a comparison made between the *Longimycelium tulufanense-*derived transglutaminase and the *S*. *mobaraensis*-derived transglutaminase in terms of cross-linking activity to peas.
[Fig. 9] Fig. 9 shows a comparison made between the *Longimycelium tulufanense-*derived transglutaminase and the *S*. *mobaraensis-*derived transglutaminase in terms of cross-linking activity to almonds.
[Fig. 10] Fig. 10 shows a comparison made between the *Longimycelium tulufanense-*derived transglutaminase and the *S*. *mobaraensis*-derived transglutaminase in terms of cross-linking activity to rye.
[Fig. 11] Fig. 11 shows a comparison made between the *Longimycelium tulufanense-*derived transglutaminase and the *S*. *mobaraensis-*derived transglutaminase in terms of cross-linking activity to wheat.
[Fig. 12] Fig. 12 shows a comparison made between the *Longimycelium tulufanense-*derived transglutaminase and the *S*. *mobaraensis-*derived transglutaminase in terms of cross-linking activity to coconuts.
[Fig. 13] Fig. 13 shows a comparison made between the *Longimycelium tulufanense-*derived transglutaminase and the *S*. *mobaraensis-*derived transglutaminase in terms of cross-linking activity to chia seeds.
[Fig. 14] Fig. 14 shows the results of the reaction of individual natural raw materials shown in Fig. 7 to Fig. 13 for 21 hours. The *Longimycelium tulufanense*-derived transglutaminase is shown on the left side of each column, whereas the *S*. *mobaraensis-*derived transglutaminase is shown on the right side of each column.
[Fig. 15] Fig. 15 shows the results of the reaction of individual natural raw materials shown in Fig. 7 to Fig. 13 at a reaction temperature of 5°C for 21 hours. The *Longimycelium tulufanense*-derived transglutaminase is shown on the left side of each column, whereas the *S*. *mobaraensis-*derived transglutaminase is shown on the right side of each column.
[Fig. 16] Fig. 16 shows a comparison of the firmness of *tofu,* when the *tofu* is produced using the *Longimycelium tulufanense*-derived transglutaminase or the *S*. *mobaraensis-*derived transglutaminase. The *Longimycelium tulufanense*-derived transglutaminase is shown on the left side of each column, whereas the *S*. *mobaraensis-*derived transglutaminase is shown on the right side of each column.
[Fig. 17] Fig. 17 shows a comparison of the firmness of soy milk yogurt, when the soy milk yogurt is produced using the *Longimycelium tulufanense-*derived transglutaminase or the *S*. *mobaraensis-derived* transglutaminase. The *Longimycelium tulufanense-derived* transglutaminase is shown on the left side of each column, whereas the *S*. *mobaraensis-*derived transglutaminase is shown on the right side of each column.

### Embodiments of Carrying out the Invention

### 1. Enzyme agent containing transglutaminase as active ingredient

The enzyme agent of the present invention contains, as an active ingredient, a transglutaminase consisting of the amino acid sequence of SEQ ID NO: 1 or an amino acid sequence equivalent to the amino acid sequence of SEQ ID NO: 1.

The term "amino acid sequence equivalent to" means an amino acid sequence that is partially different from a reference amino acid sequence (the amino acid sequence of SEQ ID NO: 1) but such a difference does not substantially influence on the function of a protein (which is herein transglutaminase activity). Accordingly, an enzyme having such an equivalent amino acid sequence catalyzes an enzyme reaction by transglutaminase. The level of the activity is not particularly limited, as long as the function as a transglutaminase can be exhibited. The activity of a transglutaminase having such an equivalent amino acid sequence is preferably the same level as or higher than the activity of the enzyme consisting of a reference amino acid sequence (having the amino acid sequence of SEQ ID NO: 1).

The amino acid sequence of SEQ ID NO: 1 is the amino acid sequence of a transglutaminase derived from *Longimycelium tulufanense* (mature body). It is to be noted that the full-length amino acid sequence of the transglutaminase derived from *Longimycelium tulufanense* is shown in SEQ ID NO: 2.

Such a "partial difference in the amino acid sequence" is generated, for example, by a deletion or substitution of one or multiple amino acids in the amino acids constituting the amino acid sequence, or by an addition or insertion of one or multiple amino acids with respect to the amino acid sequence, or by any given combination thereof. Such a partial difference in the amino acid sequence is acceptable, as long as transglutaminase activity is retained (in which some fluctuations in the activity may be acceptable). As long as these conditions are satisfied, the position in which the amino acid sequence is different is not particularly limited. In addition, such differences in the amino acid sequence may be generated in multiple sites (places).

The number of amino acids causing such a partial difference in the amino acid sequence is a number that corresponds to, for example, less than about 30%, less than about 20%, or less than about 10% of the total amino acids constituting the amino acid sequence, is preferably a number corresponding to less than about 8%, is more preferably a number corresponding to about 6%, is further preferably a number corresponding to less than about 4%, is still further preferably a number corresponding to less than about 2%, and is most preferably a number corresponding to less than about 1%. Accordingly, such an equivalent protein has an identity of, for example, about 70% or more, about 80% or more, or about 90% or more, preferably about 92% or more, more preferably about 94% or more, further preferably about 96% or more, still further preferably about 98% or more, and most preferably about 99% or more, to the reference amino acid sequence.

One typical examples of such a "partial difference in the amino acid sequence" is that a mutation (change) is generated by a deletion or substitution of 1 to 40 amino acids (preferably 1 to 30, more preferably 1 to 10, even more preferably 1 to 7, further preferably 1 to 5, and still further preferably 1 to 3 amino acids) in the amino acids constituting the amino acid sequence, or by an addition or insertion of 1 to 40 amino acids (preferably 1 to 30, more preferably 1 to 10, even more preferably 1 to 7, further preferably 1 to 5, and still further preferably 1 to 3 amino acids) with respect to the amino acid sequence, or by a combination thereof.

Preferably, an equivalent amino acid sequence is obtained by generation of a conservative amino acid substitution in amino acid residues that are not essential for transglutaminase activity. The term "conservative amino acid substitution" is used herein to mean that a certain amino acid residue is substituted with an amino acid residue having a side chain with similar properties. The amino acid residues are classified into several families depending on the side chains thereof, such as basic side chains (e.g. lysine, arginine, and histidine), acidic side chains (e.g. aspartic acid and glutamic acid), uncharged polar side chains (e.g. glycine, asparagine, glutamine, serine, threonine, tyrosine, and cysteine), non-polar side chains (e.g. alanine, valine, leucine, isoleucine proline, phenylalanine, methionine, and tryptophan), β-branched side chains (e.g. threonine, valine, and isoleucine), aromatic side chains (e.g. tyrosine, phenylalanine, tryptophan, and histidine). The conservative amino acid substitution is preferably a substitution between amino acid residues within a single family.

The identity (%) of two amino acid sequences can be determined, for example, by the following procedures. First, two sequences are aligned for optimal comparison (for example, a gap may be introduced into a first sequence to optimize the alignment with a second sequence). If a molecule (amino acid residue) at a specific position in the first sequence is identical to a molecule at the corresponding position in the second sequence, the molecules at that position are said to be identical to each other. The identity of two sequences is a function of the number of identical positions common in the two sequences (i.e., identity (%) = the number of identical positions / the total number of positions x 100), and preferably, the number and size of gaps used for optimization of the alignment are also taken into account.

Comparison of two sequences and determination of their identity can be realized using mathematical algorithms. A specific example of the mathematical algorithms usable for comparison of sequences is the algorithms that are described in Karlin and Altschul (1990) Proc. Natl. Acad. Sci. USA 87: 2264-68 and are then modified in Karlin and Altschul (1993) Proc. Natl. Acad. Sci. USA 90: 5873-77, but the example of the mathematical algorithms is not limited thereto. The identity of amino acid sequences can be obtained, for example, by blastp (protein-protein BLAST) of National Center for Biotechnology Information (NCBI). As parameters, default parameters may be used. For example, using BLOSUM62 matrix, Gap Costs may be set to be Existence: 11 and Extension: 1.

The transglutaminase that is an active ingredient of the enzyme agent of the present invention may be a part of a larger protein (for example, a fusion protein). Examples of a sequence to be added to such a fusion protein may include sequences that are useful for purification, such as multiple histidine residues, and additional sequences that ensure stability during recombinant production.

The transglutaminase of the present invention (hereinafter also referred to as "the present enzyme") can be obtained by culturing microorganisms that generate the transglutaminase (a transglutaminase-generating strain), for example, Longimycelium sp. bacteria, such as *Longimycelium tulufanense* as an example. The transglutaminase-generating strain may be either a wild-type strain or a mutant strain (such a mutant strain is obtained, for example, by ultraviolet irradiation). A specific example of the transglutaminase-generating strain may be *Longimycelium tulufanense* (NBRC107726). The *Longimycelium tulufanense* (NBRC107726) is a strain conserved at NBRC (National Institute of Technology and Evaluation, Biological Resource Center), and this strain can be furnished by going through prescribed procedures.

The present enzyme can be prepared using a culture solution and/or a cell mass of microorganisms that generate the present enzyme. The culture conditions or the culture methods are not particularly limited, as long as the present enzyme can be produced thereby. That is to say, the methods or the culture conditions that are adapted to the culture of the used microorganisms can be determined, as appropriate, under conditions in which the present enzyme is produced. Regarding the culture method, either liquid culture or solid culture may be applied, and liquid culture is preferably utilized. Taking the liquid culture as an example, the culture conditions will be described below.

The medium is not particularly limited, as long as it is a medium in which the used microorganisms can grow. Examples of the medium that can be used herein may include those to which the following substances are added: carbon sources, such as glucose, sucrose, gentiobiose, soluble starch, glycerin, dextrin, molasses, and organic acids; nitrogen sources, such as ammonium sulfate, ammonium carbonate, ammonium phosphate, ammonium acetate, or gelatin, peptone, yeast extract, corn steep liquor, casein hydrolysate, bran, and meat extract; and further, inorganic salts, such as potassium salts, magnesium salts, sodium salts, phosphates, manganese salts, iron salts and zinc salts. In order to promote the growth of the used microorganisms, vitamins, amino acids and the like may be added to the medium. The pH of the medium is adjusted to, for example, about 3 to 8, preferably about 4 to 7. The culture temperature is usually about 20°C to 40°C, preferably about 25°C to 35°C, and the microorganisms are cultured for 1 to 20 days, and preferably 3 to 10 days, under aerobic conditions. As a culture method applied herein, for example, a shaking culture method, or an aerobic deep culture method using ajar fermenter, can be utilized.

After completion of the culture performed under the above-described conditions, the enzyme of interest is recovered from the culture solution or the cell mass. When the enzyme is recovered from the culture solution, for example, the culture supernatant is filtered, centrifuged, etc. to remove insoluble matters, and thereafter, the resultant is separated and purified by appropriately combining concentration using an ultrafiltration membrane, salting-out such as ammonium sulfate precipitation, dialysis, and various types of chromatography using ion exchange resin, etc., thereby obtaining the present enzyme. On the other hand, when the enzyme is recovered from the cell mass, for example, the cell mass is crushed by a pressure treatment, an ultrasonic treatment, etc., and is then separated and purified in the same manner as that described above, so as to obtain the present enzyme. Besides, the cell mass may be previously recovered from the culture solution by filtration, a centrifugation treatment, etc., and thereafter, the above-described series of steps (crushing, separation, and purification of the cell mass) may be carried out.

Also, the present enzyme can be easily prepared by a genetic engineering technique. For example, the present enzyme can be prepared by transforming suitable host cells (for example, *Escherichia coli*) with DNA encoding the present enzyme, and then recovering the protein expressed in the transformant. The recovered protein is appropriately purified, depending on the purpose. As such, if the enzyme of interest is obtained as a recombinant protein, various modifications can be carried out thereon. For example, a DNA encoding the present enzyme and another appropriate DNA are inserted into an identical vector, and a recombinant protein is produced using the vector, so that the present enzyme consisting of a recombinant protein, in which any given peptides or proteins are linked to each other, can be obtained. In addition, modification may be carried out, so that addition of a sugar chain and/or a lipid or the processing of the N-terminus or the C-terminus may occur. By performing the aforementioned modification, simplification of the extraction and purification of a recombinant protein, addition of biological functions, etc. can be carried out.

Generally, the expression of a gene and the recovery of a gene expression product (the present enzyme) are carried out, utilizing an appropriate host-vector system, as described above. However, a cell-free synthesis system may also be utilized. Herein, the term "cell-free synthesis system (a cell-free transcription system or a cell-free transcription/translation system)" means that, not using living cells, but using a ribosome, a transcription/translation factor or the like derived from living cells (or obtained by a genetic engineering technique), from a nucleic acid (DNA or mRNA) used as a template, mRNA or a protein encoded by the nucleic acid is synthesized *in vitro.* In general, in the cell-free synthesis system, a cell extract obtained by purifying, as necessary, a cell-disintegrated solution is used. The cell extract generally comprises a ribosome, various types of factors such as an initiation factor, and various types of enzymes such as tRNA, which are necessary for protein synthesis. When a protein is synthesized, various types of amino acids, energy sources such as ATP or GTP, and other substances necessary for protein synthesis, such as creatine phosphate, are added to the aforementioned cell extract. As a matter of course, upon the synthesis of a protein, a ribosome, various types of factors, and/or various types of enzymes, and the like, which are prepared separately, may also be added to the aforementioned cell extract, as necessary.

The development of a transcription/translation system, in which various molecules (factors) necessary for protein synthesis have been reconstructed, has also been reported (Shimizu, Y. et al.: Nature Biotech., 19, 751-755, 2001). In this synthesis system, genes of 31 types of factors composed of: 3 types of initiation factors, 3 types of elongation factors, 4 types of factors associated with termination, 20 types of aminoacyl tRNA synthesis enzymes that allow each amino acid to bind to tRNA, and a methionyl tRNA formyl transfer enzyme, which constitute a protein synthesis system of bacteria, are amplified from an *Escherichia coli* genome, and then, using these amplified products, a protein synthesis system is reconstructed *in vitro.* In the present invention, such a reconstructed synthesis system may be utilized.

The term "cell-free transcription/translation system" is exchangeably used with the term "cell-free protein synthesis system," *"in vitro* translation system" or *"in vitro* transcription/translation system." In the *in vitro* translation system, RNA is used as a template to synthesize a protein. As such template RNA, total RNA, mRNA, an *in vitro* transcriptional product or the like is used. On the other hand, in the *in vitro* transcription/translation system, DNA is used as a template. The template DNA should comprise a ribosome-binding region, and preferably comprises an appropriate terminator sequence. In addition, in the *in vitro* transcription/translation system, in order to promote continuous progression of the transcription reaction and the translation reaction, conditions, in which factors necessary for each reaction are added, are established.

The purified enzyme obtained as described above is pulverized, for example, by freeze drying, vacuum drying, or spray drying, so that the enzyme can be provided in the form of powders. At that time, the purified enzyme may be previously dissolved in an acetate buffer, a phosphate buffer, a triethanolamine buffer, a Tris-HCl buffer, or a GOOD buffer. Preferably, an acetate buffer, a phosphate buffer, or a triethanolamine buffer can be used. Besides, examples of the GOOD buffer used herein may include PIPES, MES, and MOPS.

The purification degree of the enzyme is not particularly limited. In addition, the final form of the enzyme may be either a liquid or a solid (including powders).

As a result of the studies conducted by the present inventors, the properties of the *Longimycelium tulufanense*-derived transglutaminase consisting of the amino acid sequence of SEQ ID NO: 1 have been determined as follows (please refer to the after-mentioned Examples for details). Accordingly, the present enzyme can also be identified by the following enzymatic chemical properties. It is to be noted that the details of the measuring conditions, measuring procedures, etc. of transglutaminase activity necessary for evaluation of each enzymatic property will be described in the after-mentioned Examples.

### (1) Action

The present enzyme is transglutaminase.

### (2) Optimal temperature and low-temperature reactivity

When a comparison is made among 10°C, 20°C, 30°C, 40°C, 50°C, 60°C, and 70°C, the optimal temperature of the present enzyme is about 50°C.

When the enzyme activity of the present enzyme at a reaction temperature of 50°C is set to be 100%, the relative enzyme activity of the present enzyme at a reaction temperature of 20°C is 20% or more (preferably 30% or more, more preferably 40% or more, and further preferably 50% or more).

When the enzyme activity of the present enzyme at a reaction temperature of 50°C is set to be 100%, the relative enzyme activity of the present enzyme at a reaction temperature of 5°C is 10% or more (preferably 15% or more, and more preferably 20% or more); the relative enzyme activity of the present enzyme at a reaction temperature of 10°C is 10% or more (preferably 20% or more, and more preferably 30% or more); the relative enzyme activity of the present enzyme at a reaction temperature of 30°C is 40% or more (preferably 50% or more, more preferably 60% or more, and further preferably 70% or more); and the relative enzyme activity of the present enzyme at a reaction temperature of 40°C is 70% or more (preferably 80% or more, and more preferably 90% or more).

### (3) Optimal pH

When a comparison is made among pH 5, 6, 7, 8, 9, and 10, the optimal pH of the present enzyme is pH 7.

### (4) Heat stability (50°C)

After the present enzyme is subjected to a heat treatment at 50°C for 40 minutes, the enzyme activity of the present enzyme is 60% or more (preferably 70% or more, more preferably 80% or more, and further preferably 90% or more) of the enzyme activity thereof before the heat treatment.

Preferably, even if the present enzyme is subjected to a heat treatment at 50°C for 10 minutes, the activity thereof is not substantially decreased.

Preferably, even if the present enzyme is subjected to a heat treatment at 50°C for 20 minutes, the activity thereof is not substantially decreased.

Preferably, after the present enzyme is subj ected to a heat treatment at 50°C for 80 minutes, the enzyme activity of the present enzyme is 50% or more (preferably 60% or more, more preferably 70% or more, and further preferably 80% or more) of the enzyme activity thereof before the heat treatment.

Preferably, after the present enzyme is subjected to a heat treatment at 50°C for 120 minutes, the enzyme activity of the present enzyme is 40% or more (preferably 50% or more, and more preferably 60% or more) of the enzyme activity thereof before the heat treatment.

### (5) pH Stability

The enzyme activity of the present enzyme at pH 4 is 50% or more (preferably 60% or more, more preferably 70% or more, and further preferably 80% or more) of the enzyme activity thereof at pH 8.

Preferably, the enzyme activity of the present enzyme at pH 5, pH 6 and pH 7 is 80% or more (preferably 85% or more, and more preferably 90% or more) of the enzyme activity thereof at pH 8.

Preferably, the enzyme activity of the present enzyme at pH 3 is 30% or more (preferably 40% or more, more preferably 50% or more, further preferably 60% or more, and particularly preferably 70% or more) of the enzyme activity thereof at pH 8.

Preferably, the enzyme activity of the present enzyme at pH 2 is 20% or more (preferably 30% or more, more preferably 40% or more, and further preferably 50% or more) of the enzyme activity thereof at pH 8.

Preferably, the enzyme activity of the present enzyme at pH 11 is 80% or more (preferably 85% or more, and more preferably 90% or more) of the enzyme activity thereof at pH 8.

Moreover, the molecular weight of the present enzyme measured by SDS-PAGE (SDS-polyacrylamide gel electrophoresis) is 28 to 30 kDa. On the other hand, the molecular weight calculated from the amino acid sequence of SEQ ID NO: 2 is 30 kDa.

The content of the active ingredient (the present enzyme) in the enzyme agent of the present invention is not particularly limited. For example, the content of the active ingredient can be determined or adjusted, so that the transglutaminase activity per gram of the present enzyme agent becomes 0.1 U to 5000 U, preferably 1 U to 500 U, and more preferably 10 U to 300 U. It is to be noted that the unit (1 U) of the transglutaminase activity is defined as described in the after-mentioned Examples.

The enzyme agent of the present invention may also contain an excipient, a buffer agent, a suspending agent, a stabilizer, a preservative, an antiseptic, a normal saline, various types of proteins, degradation products of various types of proteins, various types of extracts, various types of salts, various types of antioxidants, cysteine, glutathione, sodium glutamate, sodium inosinate, sodium guanylate, calcinated shell calcium, silicon dioxide, and the like, as well as the active ingredient (the modified enzyme of the present invention). Examples of the excipient that can be used herein may include starch, dextrin, maltose, trehalose, lactose, D-glucose, sorbitol, D-mannitol, white sugar, and glycerol. Examples of the buffer agent that can be used herein may include phosphate, citrate, and acetate. Examples of the stabilizer that can be used herein may include propylene glycol and ascorbic acid. Examples of the preservative that can be used herein may include phenol, benzalkonium chloride, benzyl alcohol, chlorobutanol, and methylparaben. Examples of the antiseptic that can be used herein may include ethanol, benzalkonium chloride, paraoxybenzoic acid, and chlorobutanol. Examples of the protein used herein may include a soybean protein, a wheat protein, a corn protein, a milk protein, and an animal-derived protein. Examples of the extracts used herein may include a meat extract, a plant extract, and a yeast extract. Examples of the salts used herein may include chloride, phosphate, polyphosphate, pyrophosphate, citrate, lactate, and carbonate. Examples of the antioxidant used herein may include L-ascorbate and sodium hydrogen sulfite. The shape of the enzyme agent of the present invention is not particularly limited, and it is, for example, powders, granules, a liquid, a capsule, or the like.

### 2. Gene

The gene encoding the present enzyme consists of DNA encoding a protein containing the amino acid sequence of SEQ ID NO: 1. Specific examples of the present embodiment may include DNA consisting of the base sequence of SEQ ID NO: 3 and DNA consisting of the base sequence of SEQ ID NO: 4. The DNA of SEQ ID NO: 3 encodes only the amino acid sequence of a mature body (SEQ ID NO: 1), whereas the DNA of SEQ ID NO: 4 encodes a signal peptide and a pro-sequence, in addition to the mature body (the amino acid sequence of SEQ ID NO: 1).

The gene encoding the present enzyme is typically utilized in preparation of the present enzyme. According to a genetic engineering preparation method using the gene encoding the present enzyme, it is possible to obtain the present enzyme in a more homogeneous state. In addition, it is said that this method is a method preferably used in the case of preparing a large amount of the present enzyme. Besides, the intended use of the gene encoding the present enzyme is not limited to the preparation of the present enzyme. For example, the present nucleic acid can also be used as an experimental tool for elucidating the action mechanism of the present enzyme, or as a tool for designing or preparing a mutant (a modified body) of the present enzyme.

In the present description, the phrase "the gene encoding the present enzyme" means a nucleic acid for providing the present enzyme, when the nucleic acid is allowed to express. Thus, the gene encoding the present enzyme includes not only a nucleic acid having a base sequence corresponding to the amino acid sequence of the present enzyme, but also a nucleic acid formed by adding a sequence that does not encode the amino acid sequence to the aforementioned nucleic acid. Moreover, codon degeneracy is also taken into consideration.

With reference to the sequence information disclosed in the present description or the sequence listing attached therewith, the nucleic acid can be prepared in an isolated state according to a standard genetic engineering technique, a molecular biological technique, a biochemical technique, chemical synthesis, a PCR method (for example, overlap PCR), or a combination thereof.

There may be used a nucleic acid, in which when the nucleic acid is compared with the base sequence of the gene encoding the present enzyme, the function of a protein encoded thereby is equivalent, but the base sequence thereof is partially different from the base sequence of the gene encoding the present enzyme (which is hereinafter also referred to as an "equivalent nucleic acid," and the base sequence of the equivalent nucleic acid is also referred to as an "equivalent base sequence"). An example of such an equivalent nucleic acid may be DNA that encodes a protein consisting of a base sequence containing a substitution, deletion, insertion, addition or inversion of one or multiple bases, based on the base sequence of the nucleic acid encoding the present enzyme, and having enzyme activity characteristic for the present enzyme (i.e. transglutaminase activity). Substitution, deletion, etc. of base(s) may occur in multiple sites. The term "multiple" is used herein to mean, for example, 2 to 40 bases, preferably 2 to 20 bases, and more preferably 2 to 10 bases, although the number of bases is different depending on the positions or types of amino acid residues in the three-dimensional structure of a protein encoded by the nucleic acid. The equivalent nucleic acid has a sequence identity of, for example, 70% or more, 80% or more, 85% or more, or 90% or more, preferably 92% or more, more preferably 94% or more, further preferably 96% or more, still further preferably about 98% or more, and most preferably 99% or more, with respect to the base sequence serving as a reference (SEQ ID NO: 3 or SEQ ID NO: 4).

Such an equivalent nucleic acid as described above can be obtained by, for example, a restriction enzyme treatment, a treatment with exonuclease, DNA ligase, etc., introduction of a mutation according to a site-directed mutagenesis (Molecular Cloning, Third Edition, Chapter 13, Cold Spring Harbor Laboratory Press, New York) or a random mutation introduction method (Molecular Cloning, Third Edition, Chapter 13, Cold Spring Harbor Laboratory Press, New York), etc. The equivalent nucleic acid can also be obtained by other methods such as ultraviolet irradiation.

There may be used a nucleic acid having a base sequence complementary to the base sequence of the gene that encodes the present enzyme. There may be used a nucleic acid having a base sequence that is, at least, about 90%, 92%, 94%, 96%, 98% or 99% identical to the base sequence of the gene that encodes the present enzyme, or to a base sequence complementary to the base sequence of the gene that encodes the present enzyme.

There may be used a nucleic acid having a base sequence that hybridizes under stringent conditions to a base sequence complementary to the base sequence of the gene of that encodes the present enzyme or a base sequence equivalent thereto. The term "stringent conditions" is used herein to mean conditions under which, what is called, a specific hybrid is formed, but a non-specific hybrid is not formed. Such stringent conditions are known to those skilled in the art, and can be determined, for example, with reference to Molecular Cloning (Third Edition, Cold Spring Harbor Laboratory Press, New York) or Current protocols in molecular biology (edited by Frederick M. Ausubel et al., 1987). The stringent conditions may be, for example, conditions in which incubation is performed at about 50°C, using a hybridization solution (50% formamide, 10 x SSC (0.15 M NaCl, 15 mM sodium citrate, pH 7.0), 5 x Denhardt solution, 1% SDS, 10% dextran sulfate, 10 µg/ml denatured salmon sperm DNA, and 50 mM phosphate buffer (pH7.5)), and thereafter, washing is performed at about 65°C, using 0.1 x SSC and 0.1% SDS. More preferred stringent conditions may be, for example, conditions of using, as a hybridization solution, 50% formamide and 5 x SSC (0.15 M NaCl, 15 mM sodium citrate, pH 7.0), 1 x Denhardt solution, 1% SDS, 10% dextran sulfate, 10 µg/ml denatured salmon sperm DNA, 50 mM phosphate buffer (pH7.5)).

There may be used recombinant DNA containing the gene that encodes the present enzyme. The recombinant DNA is provided in the form of, for example, a vector. The term "vector" is used in the present description to mean a nucleic acid molecule capable of transporting a nucleic acid inserted in the nucleic acid molecule into a target such as a cell.

Depending on intended purpose (cloning or the expression of a protein), or taking into consideration of the type of a host cell, a suitable vector is selected. Examples of the vector having *Escherichia coli* as a host may include M13 phage or a modified body thereof, λ phage or a modified body thereof, and pBR322 or a modified body thereof (pB325, pAT153, pUC8, etc.). Examples of the vector having yeast as a host may include pYepSec1, pMFa, and pYES2. Examples of the vector having an insect cell as a host may include pAc and pVL. Examples of the vector having a mammalian cell as a host may include pCDM8 and pMT2PC.

The vector is preferably an expression vector. The term "expression vector" means a vector that is capable of introducing a nucleic acid inserted in the vector into a cell of interest (a host cell) and that is also capable of allowing the nucleic acid to be expressed in the host cell. The expression vector usually comprises a promoter sequence necessary for the expression of the inserted nucleic acid, an enhancer sequence for promoting the expression, and the like. An expression vector containing a selective marker can also be used. In the case of using such an expression vector, the presence or absence of the introduction of the expression vector (and the degree thereof) can be confirmed by utilizing the selective marker.

Insertion of the nucleic acid into the vector, insertion of a selective marker gene (if necessary), insertion of a promoter (if necessary), and the like can be carried out by using a standard recombinant DNA technique (for example, a publicly known method using restriction enzyme and DNA ligase, which can refer to Molecular Cloning, Third Edition, 1.84, Cold Spring Harbor Laboratory Press, New York).

In terms of the ease of handling, microorganisms, such as *Escherichia coli,* Filamentous fungi (*Aspergillus oryzae*)*,* Actinomycetes (Streptomyces sp., *Streptomyces lividans, Streptomyces mobaraensis*)*, Bacillus subtilis,* and budding yeast (*Saccharomyces cerevisiae*)*,* are preferably used as host cells. However, any host cells can be utilized, as long as they are capable of replicating recombinant DNA and expressing the gene of the present enzyme. In the case of utilizing a T7 promoter, the used *Escherichia coli* may be, for example, the *Escherichia coli* BL21(DE3)pLysS. In the case of not using a T7 promoter, the used *Escherichia coli* may be, for example, the *Escherichia coli* JM109. In addition, examples of the budding yeast may include the budding yeast SHY2, the budding yeast AH22, and the budding yeast INVSc1 (Invitrogen).

Microorganisms containing the recombinant DNA (i.e. a transformant) can be used. The microorganisms can be obtained by transfection or transformation using the above-described vector of the present invention. Such transfection or transformation can be carried out, for example, by a calcium chloride method (Journal of Molecular Biology (J. Mol. Biol.), Vol. 53, p. 159 (1970)), a Hanahan method (J. Mol. Biol., Vol. 166, p. 557 (1983)), an SEM method (Gene, Vol. 96, p. 23 (1990)), a method of Chung, et al. (Proceedings of the National Academy of Sciences of the USA, Vol. 86, p. 2172 (1989)), a calcium phosphate coprecipitation method, electroporation (Potter, H. et al., Proc. Natl. Acad. Sci. U.S.A. 81, 7161-7165 (1984)), and lipofection (Felgner, P. L. et al., Proc. Natl. Acad. Sci. U.S.A. 84, 7413-7417 (1984)). The aforementioned microorganisms can be utilized to produce the present enzyme.

### 3. Use of enzyme agent

The present invention further relates to a use of the enzyme agent of the present invention.

Specifically, the enzyme agent of the present invention is allowed to act on a substrate (a protein or a peptide), so that the protein or the peptide can be modified. With regard to a specific example of the use, the enzyme agent of the present invention is allowed to act on a food product or a food product material (a protein-containing food product material), so that the food product or the food product material (protein-containing food product material) can be produced. By allowing the enzyme agent of the present invention to act on a food product, the quality of the food product can be improved. According to the present invention, a food product treated with the enzyme agent of the present invention is provided. Moreover, the enzyme agent of the present invention can also be used in production of industrial materials or pharmaceutical raw materials, as well as food products or food product materials (protein-containing food product materials).

Specific examples of the food product may include, but are not particularly limited to: processed meat products (e.g., processed edible meat products such as ham and bacon; and meat paste products such as sausage, hamburger steak, and meatballs); processed marine products (e.g., fish sausage, *kamaboko* (fish paste), *chikuwa* (fish paste), fish balls, etc.); rice products (e.g., cooked white rice, rice cooked with red beans, pilaf, rice seasoned and cooked with various ingredients, rice porridge, risotto, rice balls, etc.); noodles (e.g., noodles such as *udon,* pasta, Japanese buckwheat noodles, Chinese noodles, fried noodles, instant noodles, etc.); cereal flours (e.g. wheat flour, barley flour, corn flour, buckwheat flour, etc.) and the processed foods thereof; dairy products (yogurt, cheese, butter, ice cream, etc.); *tofu* products and the processed products thereof; and vegetable protein foods (vegetable milk, vegetable yogurt (e.g. soy milk yogurt), alternative meat, etc.). Examples of the food product material may include, but are not particularly limited to, casein, soybeans, peas, chickpeas, fava beans, mung beans, almonds, oats, rye, wheat, coconuts, chia seeds, and egg. The food product materials are particularly preferably soybeans, peas, rye, wheat, coconuts, and chia seeds.

The method for producing a food product using the present enzyme is not particularly limited, and it may be, for example, a method containing the following steps (a) and (b):
(a) a step of allowing the present enzyme to act on a food product or a food product material; and
(b) a step of inactivating or removing the present enzyme contained in the obtained enzyme-treated food product or enzyme-treated food product material.

### Examples

### Example 1: Preparation of Longimycelium tulufanense-derived transglutaminase

The bacterial cells of *Longimycelium tulufanense* (NBRC107726) (obtained from the National Institute of Technology and Evaluation (NITE)) were cultured on Tryptosoya Bouillon medium (manufactured by Nissui Pharmaceutical Co., Ltd.) at 37°C for 4 days, and thereafter, the supernatant was recovered. The obtained supernatant was dialyzed against a 20 mM phosphate buffer (pH 6) according to an ordinary method. The dialyzed sample was subjected to ion exchange chromatography on a cation exchange column (SP Sepharose FF, manufactured by GE Health Care) equilibrated with the same buffer solution, and the active fraction was desalted and concentrated to obtain a roughly purified enzyme sample. Herein, the active fraction was confirmed by the method described in Example 3 later. The sample used in Example 3 was obtained by further subjecting the roughly purified enzyme sample to gel filtration using Superdex 200 HR.

### Example 2: Confirmation of sequence of Longimycelium tulufanense-derived transglutaminase

The N-terminus of the purified enzyme was analyzed, and as a result, the sequence "ATSLPPPIAPPLPRGVQSKS" was obtained. A protein was deduced from the genome information based on the obtained sequence, and as a result, the amino acid sequence of the protein was matched with the amino acid sequence of a protein with unknown function (WP_189053072.1). Using this amino acid sequence as a reference, a region containing the upstream and downstream thereof was amplified from the genome by PCR, the base sequence was then confirmed, and the amino acid sequence was then identified.

Amino acid sequence (only the sequence of a mature body) (SEQ ID NO: 1) 
ATSLPPIAPPLPRGVQSKSWSVPDYIAAWEKQHGRPMTAEERYHLARGCIGVTVV
NLDREDAPNPPLNLSFGTYQRAMEVQAALNEIVATRPSPREYAEQVRKHPALQG
VQNLVRAFPTFIDPANLHAAIFSKRFYSKQDPNWTDEQAAEMYRPNPRTGQVDM
STYRYRARPGYVNFDYGWYDEQTNNWWHANHAEPGMKVYQSTLRYYSRPLL
DFDEQVFTVAFARVA
Amino acid sequence (full-length) * The underlined portion indicates the sequence of a mature body. (SEQ ID NO: 2) 
MKKWLPRALVALFVLLGLPAGLAGTAHAAVVAHAAVATSLPPIAPPLPRGVQSKS
WSVPDYIAAWEKQHGRPMTAEERYHLARGCIGVTVVNLDREDAPNPPLNLSFGT
YQRAMEVQAALNEIVATRPSPREYAEQVRKHPALQGVQNLVRAFPTFIDPANLHA
AIFSKRFYSKQDPNWTDEQAAEMYRPNPRTGQVDMSTYRYRARPGYVNFDYG
WYDEQTNNWWHANHAEPGMKVYQSTLRYYSRPLLDFDEQVFTVAFARVA
Base sequence (mature body) (SEQ ID NO: 3) 
gccacgtcgctgccaccgatcgcaccgccgcttccccggggcgtgcagagcaagagctggtcggtgccggactacattgccg
cctgggagaagcaacacggtaggccgatgacggccgaggagcggtaccacctcgcccggggctgcatcggcgtcaccgtg
gtcaacctcgaccgggaggacgcgccgaacccgccgctcaacctgtcgttcggcacttaccagcgggccatggaagtgcagg
ccgcgctgaacgagatcgtcgcgacccggccgtcgccgcgggagtacgccgagcaggtgcgcaagcacccggcgctgcag
ggcgtgcagaacctggtgcgggcgttccccacgttcatcgacccggcgaacctgcacgccgccatcttctccaagcggttctac
tcgaagcaggacccgaactggaccgacgagcaggcggccgagatgtaccggccgaatccgcggaccggccaggtcgacat
gagcacctaccgctaccgggcgcggccgggttacgtgaacttcgactacggttggtacgacgagcagacgaacaactggtgg
cacgccaaccacgcggagccgggcatgaaggtctaccagagcacgttgcggtactactcccggccgctgctggatttcgacga
gcaggttttcaccgtggcgttcgcgcgggtcgcctga
Base sequence (full-length) (SEQ ID NO: 4)

gtgaagaagtggttgccgcgcgccctggtcgcgctgttcgtgctcctggggttacccgccggcctggccggaacggcacatgc
cgcggtcgtcgcgcacgccgccgtcgccacgtcgctgccaccgatcgcaccgccgcttccccggggcgtgcagagcaagag
ctggtcggtgccggactacattgccgcctgggagaagcaacacggtaggccgatgacggccgaggagcggtaccacctcgc
ccggggctgcatcggcgtcaccgtggtcaacctcgaccgggaggacgcgccgaacccgccgctcaacctgtcgttcggcact
taccagcgggccatggaagtgcaggccgcgctgaacgagatcgtcgcgacccggccgtcgccgcgggagtacgccgagca
ggtgcgcaagcacccggcgctgcagggcgtgcagaacctggtgcgggcgttccccacgttcatcgacccggcgaacctgca
cgccgccatcttctccaagcggttctactcgaagcaggacccgaactggaccgacgagcaggcggccgagatgtaccggccg
aatccgcggaccggccaggtcgacatgagcacctaccgctaccgggcgcggccgggttacgtgaacttcgactacggttggta
cgacgagcagacgaacaactggtggcacgccaaccacgcggagccgggcatgaaggtctaccagagcacgttgcggtacta
ctcccggccgctgctggatttcgacgagcaggttttcaccgtggcgttcgcgcgggtcgcctga

### Example 3: Confirmation of molecular weight of Longimycelium tulufanense-derived transglutaminase

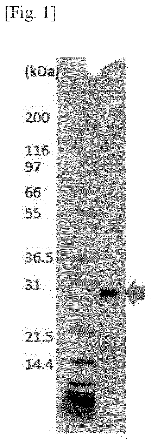

A *Longimycelium tulufanense*-derived transglutaminase (gel filtration-purified sample) was electrophoresed on NuPAGE (registered trademark) Novex 4-12% Bis-Tris Gel w/MES, and was then stained with Coomassie (registered trademark) Blue R-250. Mark12^{™} Unstained Standard was used as a molecular weight marker. The results are shown in Fig. 1. A band of the present enzyme was confirmed in a position of 28 to 30 kDa.

### Example 4: Confirmation of various properties of enzyme

### (Enzymes used)

*S*. *mobaraensis-*derived transglutaminase (control product)
Longimycelium-derived transglutaminase (the present invention)

### (Method of measuring activity)

The enzyme was dissolved in 200 mM Tris-HCl (pH6.0) containing 0.4% cysteine, and was then treated at 30°C for 1 hour. Thereafter, the obtained solution was diluted to an appropriate concentration with 200 mM Tris-HCl (pH6.0) (sample solution). After that, 100 µL of a substrate solution (R-1) was added to and mixed with 10 µL of the sample solution, and the obtained mixture was then reacted at 37°C for 10 minutes. Thereafter, 100 µL of a chromogenic solution (R-2) was added to the reaction mixture, so that the reaction was terminated, and at the same time, an Fe complex was formed. Thereafter, the absorbance at 525 nm was measured. As a control, using a previously heat-inactivated enzyme solution, the reaction was carried out in the same manner as that described above, and the absorbance of the reaction solution was then measured. A difference in absorbance from the sample solution was obtained. A calibration curve was separately prepared using L-glutamic acid-γ-monohydroxamic acid instead of the enzyme solution, and the amount of hydroxamic acid produced was determined from the difference in absorbance. The enzyme activity of producing 1 µmol of hydroxamic acid per minute was defined to be 1 unit (1 U).

### (Substrate solution (R-1))

2.42 g of 2-Amino-2-hydroxymethyl-1.3-propanediol, 0.70 g of hydroxylammonium hydrochloride, 0.31 g of reduced glutathione, and 1.01 g of Z-Gln-Gly (benzyloxycarbonyl-L-glutaminylglycine) were dissolved in distilled water to a total amount of 100 mL (pH 6.0).

### (Substrate solution (R-2))

30 mL of a 3 M hydrochloric acid solution, 30 mL of a 12% trichloroacetic acid solution, and 30 mL of a 5% iron(III) chloride solution were mixed with one another.

### (Method of confirming properties)

### < Method of confirming optimal temperature >

For the measurement of the optimal temperature, the temperature was changed to each temperature, and the activity was then measured at each temperature.

### < Method of confirming optimal pH >

The substrate solution to be used in the measurement of activity was adjusted with a 20 mM Britton-Robinson buffer (pH 5-10), and the activity was then measured.

### < Method of confirming heat stability >

The sample was subjected to a heat treatment at 50°C, and the residual activity was then measured.

### < Method of confirming pH stability >

The enzyme sample was 2-fold diluted with a 20 mM Britton-Robinson buffer (pH 2-10), and was then treated at 37°C for 1 hour. Thereafter, the residual activity was then measured.

### (Results)

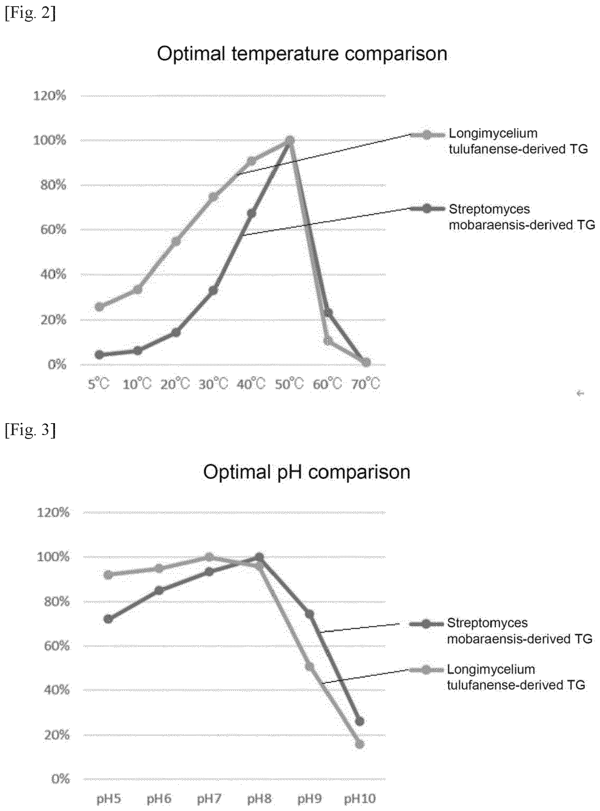

The measurement results of the optimal temperature are shown in Table 1 and Fig. 2.

The measurement results of the optimal pH are shown in Table 2 and Fig. 3.

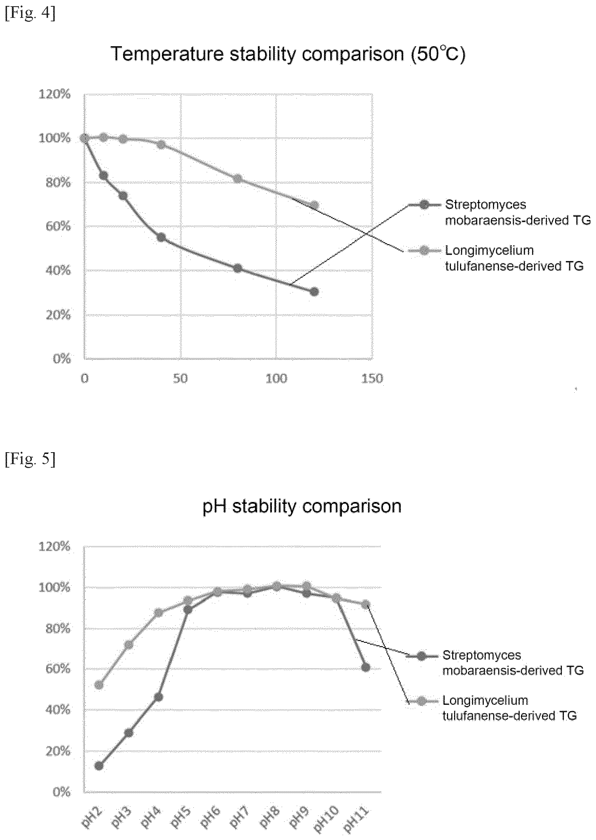

The measurement results of the heat stability are shown in Table 3 and Fig. 4.

The measurement results of the pH stability are shown in Table 4 and Fig. 5.

**[Table 1]**

| Relative values obtained when the activity at optimal temperature (50°C) is set as 100% | | | | | | | | |
|---|---|---|---|---|---|---|---|---|
| | 5°C | 10°C | 20°C | 30°C | 40°C | 50°C | 60°C | 70°C |
| *Streptomyces mobaraensis-*derived TG | 4% | 6% | 14% | 33% | 67% | 100% | 23% | 0% |
| *Longimycelium tulufanense-*derived TG | 26% | 33% | 55% | 75% | 91% | 100% | 10% | 1% |

**[Table 2]**

| Relative values obtained when the activity at optimal pH is set as 100% | | | | | | |
|---|---|---|---|---|---|---|
| | pH 5 | pH 6 | pH 7 | pH 8 | pH 9 | pH 10 |
| *Streptomyces mobaraensis*-derived TG | 72% | 85% | 93% | 100% | 74% | 26% |
| *Longimycelium tulufanense-*derived TG | 92% | 95% | 100% | 96% | 51% | 16% |

**[Table 3]**

| Residual activity over time upon treatment at 50°C | | | | | | |
|---|---|---|---|---|---|---|
| | 0 min | 10 min | 20 min | 40 min | 80 min | 120 min |
| *Streptomyces mobaraensis-*derived TG | 100% | 83% | 74% | 55% | 41% | 30% |
| *Longimycelium tulufanense-*derived TG | 100% | 101% | 100% | 97% | 82% | 69% |

**[Table 4]**

| Residual activity after pH treatment | | | | | |
|---|---|---|---|---|---|
| | pH 2 | pH 3 | pH 4 | pH 5 | pH 6 |
| *Streptomyces mobaraensis-*derived TG | 13% | 29% | 47% | 89% | 98% |
| *Longimycelium tulufanense-*derived TG | 52% | 72% | 88% | 93% | 98% |

| | pH 7 | pH 8 | pH 9 | pH 10 | pH 11 |
|---|---|---|---|---|---|
| *Streptomyces mobaraensis-*derived TG | 97% | 100% | 97% | 95% | 61% |
| *Longimycelium tulufanense-*derived TG | 99% | 101% | 101% | 95% | 92% |

### (Consideration)

As shown in Tables 1 to 4 and Fig. 2 to Fig. 5, it was confirmed that the transglutaminase of the present invention has high reactivity at a low temperature and high heat stability, when compared with the existing transglutaminase.

### Example 5: Confirmation of polymer-cross-linking activity

It was confirmed using casein as a substrate that a high-molecular-weight protein is cross-linked by a transglutaminase.

### (Method)

10 µL of a purified enzyme solution (0.1 mg/mL) was mixed into 20 µl of a 5% casein solution (prepared with a 50 mM phosphate buffer (pH 7)), and the obtained mixture was then reacted at 5°C and 37°C. Sampling was carried out timely, followed by 50-fold dilution, and thereafter, an SDS-PAGE sample buffer was added to the resultant. The obtained mixture was boiled, so that the reaction was terminated. Thereafter, the reaction mixture was subjected to SDS-PAGE.

### (Results)

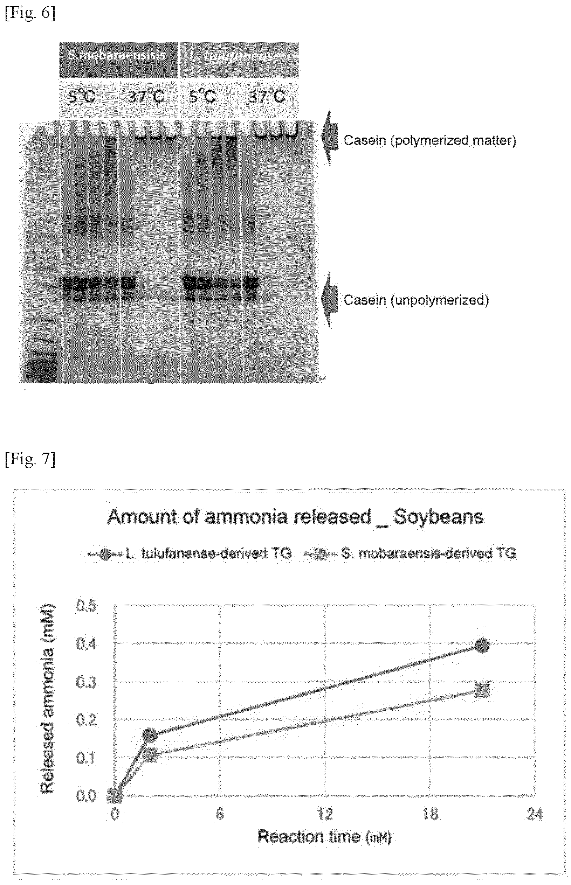

The results obtained by confirming the cross-linking reactivity of the transglutaminase are shown in Fig. 6.

In Fig. 6, the results of SDS-PAGE performed on the samples for the reaction times of 0 hour, 1 hour, 3 hours and 5 hours are shown from the left.

### (Consideration)

It was confirmed from the results shown in Fig. 6 that the *L. tulufanense*-derived enzyme is highly active even at a low temperature for the same weight. The transglutaminase of the present invention was demonstrated to be a transglutaminase with high low-temperature reactivity.

### Example 6: Confirmation of cross-linking activity on natural raw materials (SDS-PAGE)

It was confirmed using the following raw materials as substrates that natural raw material-derived proteins are cross-linked by a transglutaminase.

**Table 5**

| Substrate | Raw Material |
|---|---|
| Casein | Casein, Bovine Milk (Merck) |
| Soybeans | Soypro (J-OII, MILLS) |
| Peas | Propulse (ORGANO FOODTECH CORPORATION) |
| Chickpeas | CP-AC (ORGANO FOODTECH CORPORATION) |
| Fava beans | FP-AC (ORGANO FOODTECH CORPORATION) |
| Mung beans | MP-AC (ORGANO FOODTECH CORPORATION) |
| Almonds | Almond Powder (Pioneer-Kikaku) |
| Oats | PrOatein (Lantmännen Oats) |
| Rye | Fine ground rye flour (Petit Pas) |
| Wheat | VITEN (Roquette) |
| Coconuts | Organic Coconut Flour (Alishan ) |
| Chia seeds | Orplus SC (ORGANO FOODTECH CORPORATION) |
| Egg | Egg white (Sigma-Aldrich) |

### (Method)

10 µL of a purified enzyme solution at each concentration (0.01 mg/mL, 0.1 mg/mL, and 1 mg/mL) was mixed into 90 µL of a 1% substrate solution (prepared with a 50 mM phosphate buffer (pH 7)), and the obtained mixture was then reacted at 5°C and 37°C overnight. An SDS-PAGE sample buffer was added to the reaction mixture, and the obtained mixture was then boiled, so that the reaction was terminated. Thereafter, the reaction mixture was subjected to SDS-PAGE.

### (Results)

The cross-linking reactivity of the transglutaminase could be confirmed in the case of using all natural raw materials as substrates. It was demonstrated that the transglutaminase of the present invention is able to catalyze cross-linking reactions for various natural raw materials.

### Example 7: Confirmation of cross-linking activity on natural raw materials (quantification of amount of ammonia released)

For the purpose of quantifying a cross-linking reaction on proteins derived from natural raw materials, the amount of ammonia released by the cross-linking reaction was quantified. The raw materials shown in the table of Example 6 were used as substrates.

### (Method)

0.15 mL of a 1 mg/mL purified enzyme solution was mixed into 1.35 mL of a 1% substrate solution (prepared with a 50 mM phosphate buffer (pH 7)), and the obtained mixture was then reacted at 5°C and 37°C for 2 and 21 hours. After completion of the reaction, an equal amount of trichloroacetic acid was added to the reaction mixture, so that the reaction was terminated. The amount of ammonia released was measured according to Ammonia Test·Waco. The amount of ammonia released before the reaction (0 hour) was set as 0 mM, and the amount of ammonia released over time was quantified.

### (Results)

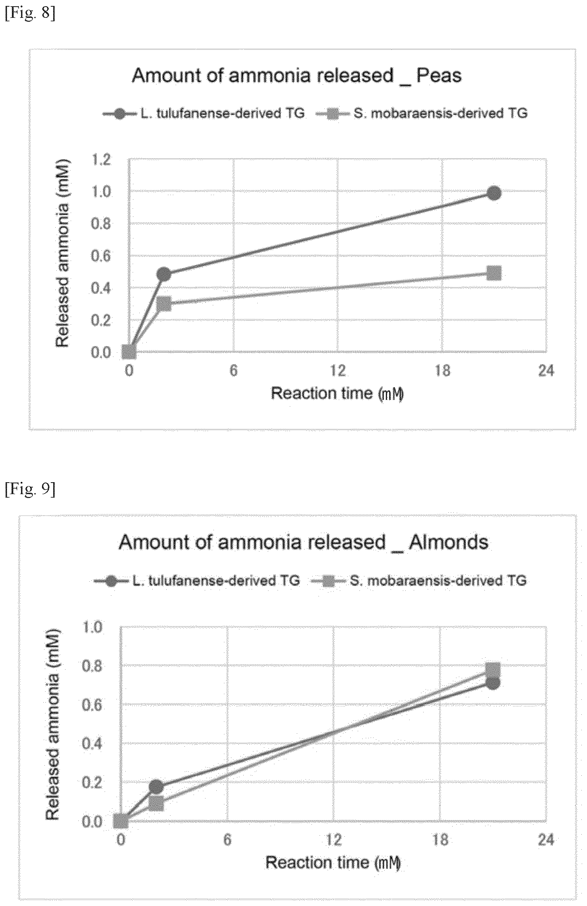

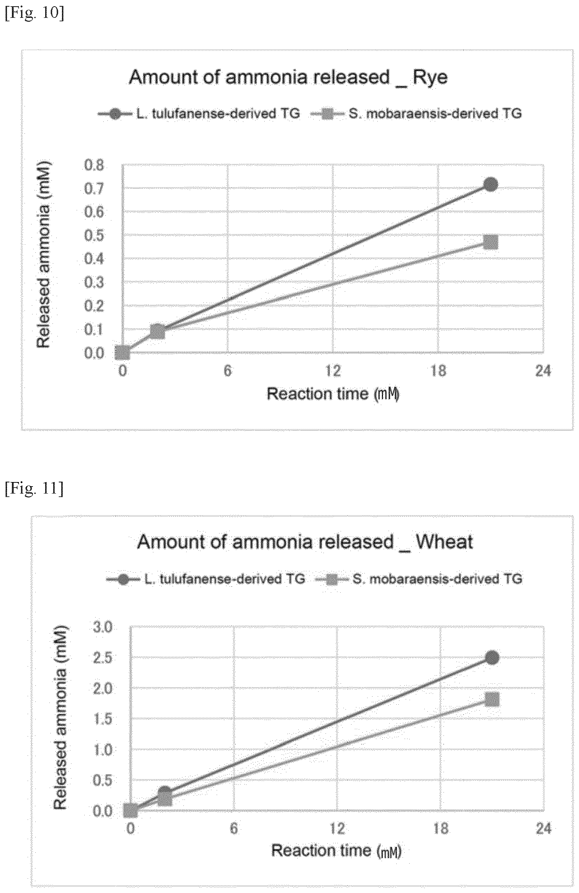

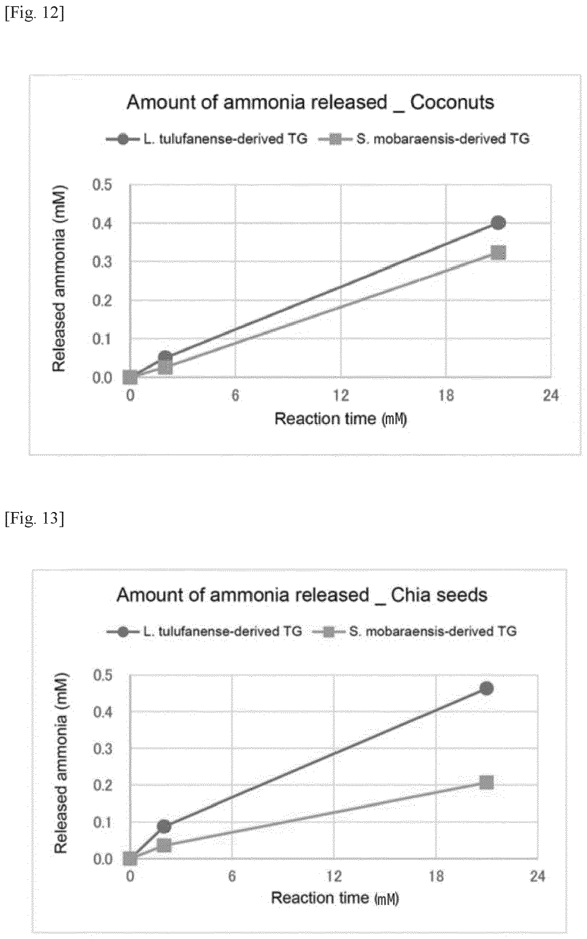

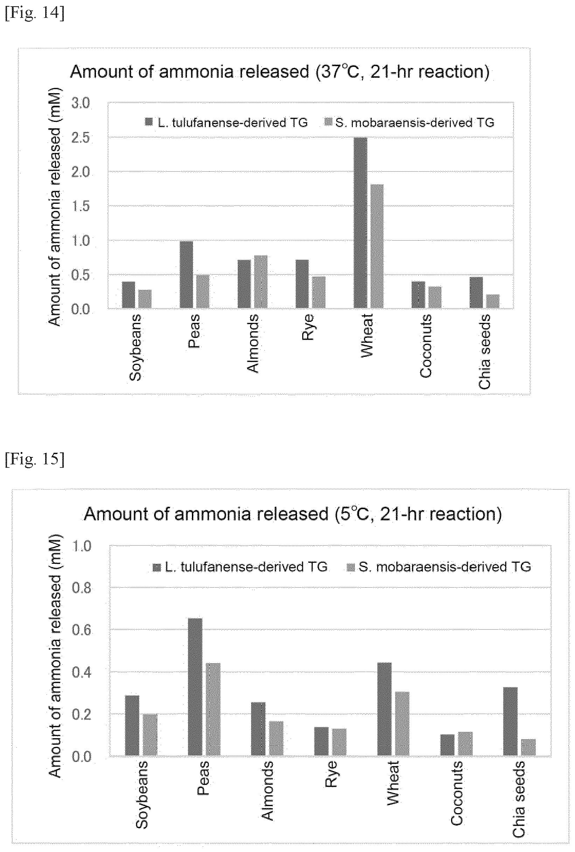

The measurement results are shown in Figs. 7 to 13. Moreover, the results of individual natural raw materials shown in Fig. 7 to Fig. 13 upon the reaction for 21 hours are shown in Fig. 14.

An increase in the amount of ammonia released over time could be confirmed in the case of using all natural raw materials as substrates. In particular, the amount of ammonia released by the present enzyme in the case of using soybeans, peas, rye, wheat, coconuts, and chia seeds was larger than the amount of ammonia released by the existing transglutaminase, and thus, it was suggested that the present enzyme be likely to have high cross-linking reactivity.

Furthermore, the results of individual natural raw materials shown in Fig. 7 to Fig. 13 that were reacted at a reaction temperature of 5°C for 21 hours are shown in Fig. 15. In the case of using all natural raw materials as substrates, the amount of ammonia released by the present enzyme is equal to or higher than the amount of ammonia released by the existing transglutaminase, and thus, it was suggested that the present enzyme be likely to have high cross-reactivity at a low temperature.

### Example 8: Production of tofu

The effects of the present enzyme on *tofu* production were confirmed.

### (Method)

20 mL of Soy milk (organic soy milk, unadjusted; SUJAHTAMEIRAKU; protein content: 5%) was treated at 55°C for 5 minutes. Thereafter, 0.6 mL of a 20% magnesium chloride hexahydrate solution, and 1 mL of a purified enzyme solution that had been diluted so that the enzyme amount per gram of soy milk protein became 25 µg, 50 µg, 100 µg or 250 µg, were added to the soy milk, and the obtained mixture was then stirred for 5 seconds. After the mixture had been reacted at 55°C for 1 hour, the reaction mixture was preserved at 4°C for 2 hours, so as to prepare *tofu.* As a control, another *tofu* was prepared without addition of the enzyme. The firmness (Firmness (N); which is specifically compressive strength) of the obtained *tofu* was measured using a rheometer (COMPAC-100II, Sun Scientific Co., Ltd.). The measurement conditions were set to be Mode: 20, adaptor: No. 13, repeating number: 1, and pushing distance: 5 mm.

### (Results)

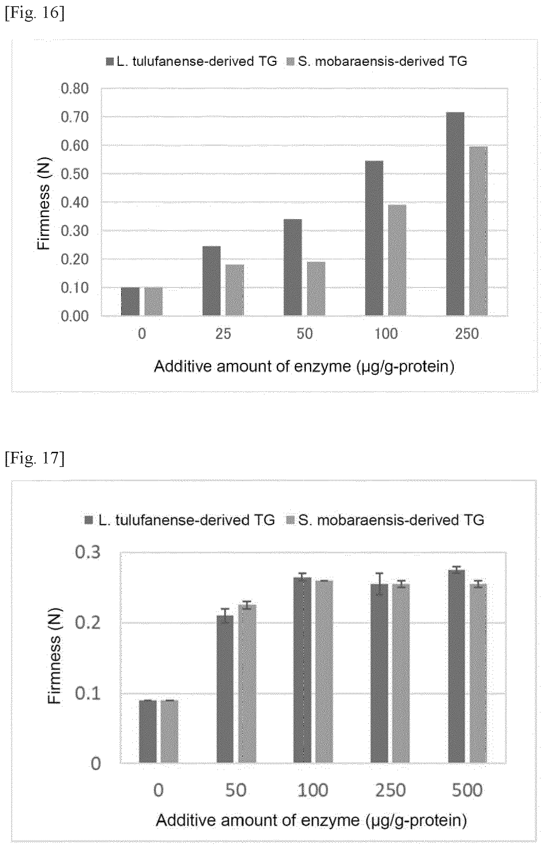

The measurement results are shown in Fig. 16.

The improvement of the firmness of the *tofu* by using the present enzyme could be confirmed. In addition, it could also be confirmed that the present enzyme has higher effects than the existing transglutaminase.

### Example 9: Production of soy milk yogurt

The effects of the present enzyme on production of soy milk yogurt were confirmed.

### (Method)

50 mL of Soy milk (organic soy milk, unadjusted; SUJAHTAMEIRAKU; protein content: 5%) was added into a 100-mL beaker, and 0.3 g of seed bacteria (King's Yogurt Seed Bacteria, Ota's Isan Co.,Ltd) (cremoris bacteria BRF (DM-1 and DM-2 strains) and Thermophilus bacteria (KI-1 strain)) were then added to the soy milk. 1 mL of a purified enzyme solution was diluted, so that the enzyme amount per gram of soy milk protein became 50 µg, 100 µg or 250 µg, and the thus diluted enzyme solution was ten added to the above mixture, and thereafter, the obtained mixture was fully stirred. The beaker was covered with a wrap film, and was then left at rest at 28°C for 20 hours, and thereafter, it was preserved at 4°C, so as to prepare soy milk yogurt. As a control, another *tofu* was prepared without addition of the enzyme. The firmness (Firmness (N); which is specifically compressive strength) of the obtained *tofu*-was measured using a rheometer (COMPAC-100II, Sun Scientific Co., Ltd.). The measurement conditions were set to be Mode: 20, adaptor: No. 13, repeating number: 1, and pushing distance: 5 mm.

### (Results)

The measurement results are shown in Fig. 17.

The improvement of the firmness of the soy milk yogurt by using the present enzyme could be confirmed.

### Industrial Applicability

The enzyme agent of the present invention contains, as an active ingredient, a transglutaminase having high reactivity at a low temperature. Therefore, the enzyme agent of the present invention is suitably used in the fields of food products and medical uses, and thus, the present enzyme agent has a high value in industrial application.

The present invention is not limited in any way to the above-described embodiments and examples of the present invention. Various modifications are also included in the present invention, in such a range that those skilled in the art can readily conceive of them, and that the modifications are not deviated from the scope of the claims. The contents of the study papers, laid-open patent publications, patent publications, etc., which are explicitly mentioned in the present description, are cited herein by reference in their entirety.

## Claims

1. An enzyme agent containing, as an active ingredient, a transglutaminase consisting of an amino acid sequence having a sequence identity of 90% or more to the amino acid sequence of SEQ ID NO: 1.

2. The enzyme agent according to claim 1, wherein the transglutaminase is derived from Longimycelium sp.

3. An enzyme agent containing, as an active ingredient, a transglutaminase derived from Longimycelium sp., in which the transglutaminase has a molecular weight of 28 to 30 kDa as measured by SDS-PAGE, and an enzyme activity of the transglutaminase at 20°C is 20% or more of the enzyme activity thereof at 50°C.

4. The enzyme agent according to claim 3, wherein an optimal pH is pH 6.5 to 7.5.

5. The enzyme agent according to claim 3 or 4, wherein the enzyme activity after a heat treatment at 50°C for 40 minutes is 60% or more of the enzyme activity before the heat treatment.

6. The enzyme agent according to any one of claims 3 to 5, wherein the enzyme activity at pH 4 is 50% or more of the enzyme activity at pH 8.

7. A method for modifying a protein or a peptide, containing allowing the enzyme agent according to any one of claims 1 to 6 to act on a substrate.

8. A method for producing a food product, containing allowing the enzyme agent according to any one of claims 1 to 6 to act on a food product.

9. A food product treated with the enzyme agent according to any one of claims 1 to 6.

10. A method for producing a transglutaminase consisting of an amino acid sequence having a sequence identity of 90% or more to the amino acid sequence of SEQ ID NO: 1, containing
culturing Longimycelium sp. bacteria.

11. A method for producing a transglutaminase, containing
culturing a *Longimycelium tulufanense* NBRC107726 strain.
